(19) **Europäisches Patentamt**

**European Patent Office**

**Office européen des brevets**

(11) **EP 1 294 394 B1**

(12) **EUROPEAN PATENT SPECIFICATION**

(45) Date of publication and mention
of the grant of the patent:
**12.04.2006 Bulletin 2006/15**

(51) Int Cl.:
**A61K 38/55** (2006.01)    **A61P 7/02** (2006.01)

(21) Application number: **01938923.8**

(22) Date of filing: **06.06.2001**

(86) International application number:
**PCT/SE2001/001288**

(87) International publication number:
**WO 2001/095931 (20.12.2001 Gazette 2001/51)**

(54) **A COMBINATION PRODUCT COMPRISING MELAGATRAN AND A FACTOR Xa INHIBITOR**

EIN KOMBINATIONSPRODUKT ENTHALTEND MELAGATRAN UND EINEN FAKTOR Xa INHIBITOR

PRODUIT COMBINE CONTENANT DU MELAGATRAN ET UN INHIBITEUR DU FACTEUR Xa

(84) Designated Contracting States:
**AT BE CH CY DE DK ES FI FR GB GR IE IT LI LU MC NL PT SE TR**
Designated Extension States:
**SI**

(30) Priority: **10.06.2000 GB 0014136**

(43) Date of publication of application:
**26.03.2003 Bulletin 2003/13**

(73) Proprietor: **AstraZeneca AB**
**151 85 Södertälje (SE)**

(72) Inventor: **MATTSSON, Christer**
**S-431 83 Mölndal (SE)**

(56) References cited:
**WO-A1-97/39770**

• **TOMIHISA KAWASAKI ET AL.: 'Effect of a synthetic factor Xa inhibitor, YM-60828, on blood vessel patency in combination with a thrombolytic agent and on blood loss from the operation site in a rat model of arterial thrombosis' THROMB. HAEMOST. vol. 79, 1998, pages 859 - 864, XP002948830**

**Description**

**Field of the Invention**

This invention relates to a new combination of pharmaceutically-active compounds.

**Background and Prior Art**

Blood coagulation is the key process involved in both haemostasis (i.e. the prevention of blood loss from a damaged vessel) and thrombosis (i.e. the formation of a blood clot in a blood vessel, sometimes leading to vessel obstruction).

Coagulation is the result of a complex series of enzymatic reactions.

One of the ultimate steps in this series of reactions is the conversion of the proenzyme prothrombin to the active enzyme thrombin.

Thrombin is known to play a central role in coagulation. It activates platelets, leading to platelet aggregation, converts fibrinogen into fibrin monomers, which polymerise spontaneously into fibrin polymers, and activates factor XIII, which in turn crosslinks the polymers to form insoluble fibrin. Furthermore, thrombin activates factor V and factor VIII leading to a "positive feedback" generation of thrombin from prothrombin.

International patent application WO 94/29336 discloses a group of thrombin-inhibiting compounds, including HOOC-$CH_2$-($R$)Cgl-Aze-Pab-H (in which Cgl represents cyclohexylglycine, Aze represents $S$-azetidine-2-carboxylic acid and Pab-H represents 4-aminomethylamidinobenzene), which is also known as melagatran (see Example 1 of WO 94/29336). International Patent Application WO 97/23499 discloses prodrugs of *inter alia* melagatran.

Factor Xa is one of a cascade of proteases involved in the process of blood coagulation. Factor Xa is the preceding protease, which cleaves prothrombin to generate thrombin.

Certain compounds are known to possess Factor Xa inhibitory properties and the field has been reviewed by R.B. Wallis, *Current Opinion in Therapeutic Patents,* 1993, 1173-1179. It is known that two proteins, one known as antistatin and the other known as tick anticoagulant protein (TAP), are specific Factor Xa inhibitors which possess antithrombotic properties in various animal models of thrombotic disease.

It is also known that certain non-peptidic compounds possess Factor Xa inhibitory properties.

None of the above-mentioned documents disclose or suggest the administration of melagatran or a pharmaceutically-acceptable derivative thereof in conjunction with a Factor Xa inhibitor. We have now found, surprisingly, that administration of just such a combination gives rise to a notable synergistic anticoagulant effect.

**Disclosure of the Invention**

According to a first aspect of the invention there is provided a combination product comprising:

(A) melagatran or a salt, solvate, or prodrug thereof, wherein the prodrug is of the formula

$R^1O_2C$-$CH_2$-($R$)Cgl-Aze-Pab-OH,

wherein $R^1$ represents $C_{1-10}$ alkyl or benzyl and the OH group replaces one of the amidino hydrogens in Pab; and
(B) a Factor Xa inhibitor or a salt or solvate thereof,

wherein each of components (A) and (B) is formulated in admixture with a pharmaceutically-acceptable adjuvant, diluent or carrier.

The combination product according to the invention provides for the administration of melagatran (or salt, solvate or prodrug thereof) in conjunction with a Factor Xa inhibitor (or salt or solvate thereof), and may thus be presented either as separate formulations, wherein at least one of those formulations comprises melagatran and at least one comprises Factor Xa inhibitor, or may be presented (i.e. formulated) as a combined preparation (i.e. presented as a single formulation including melagatran and Factor Xa inhibitor).

Thus, there is further provided:

(1) a pharmaceutical formulation including melagatran or a salt, solvate, or prodrug thereof, wherein the prodrug is of the formula

$R^1O_2C$-$CH_2$-($R$)Cgl-Aze-Pab-OH,

wherein $R^1$ represents $C_{1-10}$ alkyl or benzyl and the OH group replaces one of the amidino hydrogens in Pab, and a Factor Xa inhibitor or a salt or solvate thereof, in admixture with a pharmaceutically-acceptable adjuvant, diluent or carrier (which formulation is hereinafter referred to as a "combined preparation"); and

(2) a kit of parts comprising components:

(a) a pharmaceutical formulation including melagatran or a salt, solvate, or prodrug thereof, wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab, in admixture with a pharmaceutically-acceptable adjuvant, diluent or carrier: and
(b) a pharmaceutical formulation including a Factor Xa inhibitor or a salt or solvate thereof, in admixture with a pharmaceutically-acceptable adjuvant, diluent or carrier,

which components (a) and (b) are each provided in a form that is suitable for administration in conjunction with the other.
According to a further aspect of the invention, there is provided a method of making a kit of parts as defined above, which method comprises bringing a component (a), as defined above, into association with a component (b), as defined above, thus rendering the two components suitable for administration in conjunction with each other.
By bringing the two components "into association with" each other, we include that components (a) and (b) of the kit of parts may be:

(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Thus, there is further provided a kit of parts comprising:

(I) one of components (a) and (b) as defined herein; together with (II) instructions to use that component in conjunction with the other of the two components.

The kits of parts described herein may comprise more than one formulation including an appropriate quantity/ dose of melagatran salt, solvate or prodrug thereof, and/or more than one formulation including an appropriate quantity/ dose of Factor Xa inhibitor, salt or solvate thereof, in order to provide for repeat dosing. If more than one formulation (comprising either active compound) is present, such formulations may be the same, or may be different in terms of the dose of melagatran (or salt, solvate or prodrug or Factor Xa inhibitor (or salt or solvate), chemical composition and/or physical form.
A further aspect of the invention provides the use of a pharmaceutical formulation including melagatran (or a salt, solvate, or prodrug thereof, wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab, and a Factor Xa inhibitor (or a salt or solvate thereof), in admixture with a pharmaceutically-acceptable adjuvant, diluent or carrier for the manufacture of a medicament for a method of treatment of a condition where anticoagulant therapy is indicated.
A further aspect of the invention provides the use of

(a) a pharmaceutical formulation including melagatran or a salt, solvate, or prodrug thereof, wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab, in admixture with a pharmaceutically-acceptable adjuvant diluent or carrier; in conjunction with
(b) a pharmaceutical formulation including a Factor Xa inhibitor or a salt or solvate thereof, in admixture with a pharmaceutically-acceptable adjuvant, diluent or carrier, for the manufacture of a medicament for a method of treatment of a condition where anticoagulant therapy is indicated (by which we mean where anticoagulation is required.

For the avoidance of doubt, as used herein, the term "treatment" includes therapeutic and/or prophylactic treatment.

With respect to the kits of parts as described herein, by "administration in conjunction with", we include that respective formulations comprising melagatran (or salt, solvate or prodrug thereof) and Factor Xa inhibitor (or salt or solvate thereof) are administered, sequentially, separately and/or simultaneously, over the course of treatment of the relevant condition, which condition may be acute or chronic.

Thus, in respect of the combination product according to the invention, the term "administration in conjunction with" includes that the two components of the combination product (melagatran/salt, solvate or prodrug and Factor Xa inhibitor/salt or solvate) are administered (optionally repeatedly), either (in the case of a combined preparation) together, or (in the case of a kit of parts) sufficiently closely in time, to enable a beneficial effect for the patient, that is greater, over the course of the treatment of the relevant condition, than if either a formulation comprising melagatran/salt, solvate or prodrug or a formulation comprising Factor Xa inhibitor/salt or solvate, are administered (optionally repeatedly) alone, in the absence of the other component, over the same course of treatment. Determination of whether a combination provides a greater beneficial effect in respect of, and over the course of treatment of, a particular condition, will depend upon the condition to be treated or prevented, but may be achieved routinely by the skilled person.

Further, in the context of a kit of parts according to the invention, the term "in conjunction with" includes that one or other of the two formulations may be administered (optionally repeatedly) prior to, after, and/or at the same time as, administration with the other component. When used in this context, the terms "administered simultaneously" and "administered at the same time as" include that individual doses of melagatran (or salt, solvate or prodrug thereof) and Factor Xa inhibitor (or salt or solvate thereof) are administered within 48 hours (e.g. 24 hours) of each other.

"Pharmaceutically-acceptable salts and solvates" of melagatran and Factor Xa inhibitors includes pharmaceutically-acceptable non-toxic organic or inorganic acid addition salts. It will be appreciated that the term further includes derivatives that have the same biological function and/or activity as melagatran, or the Factor Xa inhibitor, as appropriate. "Prodrugs" of melagatran include any composition of matter that, following oral or parenteral administration, is metabolised *in vivo* to form melagatran in an experimentally-detectable amount, and within a predetermined time (e.g. within a dosing interval of between 6 and 24 hours (i.e. once to four times daily)). For the avoidance of doubt, the term "parenteral" adminstration includes all forms of adminstration other than oral administration.

Factor Xa inhibitors that may be used in the combination products according to the invention include those described in *Current Opinion in Therapeutic Patents,* 1993, 1173-1179 and in international patent applications WO 00/20416, WO 00/12479, WO 00/09480, WO 00/08005, WO 99/64392, WO 99/62904, WO 99/57096, WO 99/52895, WO 99/50263, WO 99/50257, WO 99/50255, WO 99/50254, WO 99/48870, WO 99/47503, WO 99/42462, WO 99/42439, WO 99/40075, WO 99/37304, WO 99/36428, WO 99/33805, WO 99/33800, WO 99/32477, WO 99/32454, WO 99/31092, WO 99/26941, WO 99/26933, WO 99/26932, WO 99/26919, WO 99/26918, WO 99/25720, WO 99/16751, WO 99/16747, WO 99/12935, WO 99/12903, WO 99/11658, WO 99/11617, WO 99/10316, WO 99/07732, WO 99/07731, WO 99/05124, WO 99/00356, WO 99/00128, WO 99/00127, WO 99/00126, WO 99/00121, WO 98/57951, WO 98/57937, WO 98/57934, WO 98/54164, WO 98/46591, WO 98/31661, WO 98/28282, WO 98/28269, WO 98/25611, WO 98/24784, WO 98/22483, WO 98/16547, WO 98/16525, WO 98/16524, WO 98/16523, WO 98/15547, WO 98/11094, WO 98/07725, WO 98/06694, WO 98/01428, WO 97/48706, WO 97/46576, WO 97/46523, WO 97/38984, WO 97/30971, WO 97/30073, WO 97/29067, WO 97/24118, WO 97/23212, WO 97/21437, WO 97/08165, WO 97/05161, WO 96/40744, WO 96/40743, WO 96/40679, WO 96/40100, WO 96/38421, WO 96/28427, WO 96/19493, WO 96/16940, WO 95/28420, WO 94/13693, WO 00/24718, WO 99/55355, WO 99/51571, WO 99/40072, WO 99/26926, WO 98/51684, WO 97/48706, WO 97/24135, WO 97/11693, WO 00/01704, WO 00/71493, WO 00/71507, WO 00/71508, WO 00/71509, WO 00/71511, WO 00/71512, WO 00/71515, WO 00/71516, WO 00/13707, WO 00/31068, WO 00/32590, WO 00/33844, WO 00/35859, WO 00/35886, WO 00/38683, WO 00/39087, WO 00/39092, WO 00/39102, WO 00/39108, WO 00/391I1, WO 00/39117, WO 00139118, WO 00/39131, WO 00/40548, WO 00/40571, WO 00/40583, WO 00/40601, WO 00/47207, WO 00/47553, WO 00/47554, WO 00/47563, WO 00/47578, WO 00/51989, WO 00/53264, WO 00/59876, WO 00/59902, WO 00/71510, WO 00/76970, WO 00/76971, WO 00/78747, WO 01/02356, WO 01/02397, WO 01/05784, WO 01/09093, WO 01/12600, WO 01/19788, WO 01/19795, WO 01/19798, WO 93/15756, WO 94/17817, WO 95/29189, WO 96/18644, WO 96/20689, WO 96/39380, WO 97/22712, WO 97/36580, WO 97/36865, WO 97/48687, WO. 98/09987, WO 98/46626, WO 98/46627, WO 98/46628, WO 98/54132, WO 99/07730, WO 99/33458, WO 99/37643 and WO 99/64446; in US patents Nos. 6,034,093, 6,020,357, 5,994,375, 5,886,191, 5,849,519, 5,783,421, 5,731,315, 5,721,214, 5,693,641, 5,633,381, 5,612,378, 6,034,127, 5,670,479, 5,658,939, 5,658,930, 5,656,645, 5,656,600, 5,639,739, 5,741,819, 6,057,342, 6,060,491, 6,080,767, 6,087,487, 6,140,351 and 5,646,165; in Japanese patent applications Nos. JP 99152269, JP 10017549, JP 10001467, JP 98017549, JP 00178243, JP 11140040, JP 12143623, JP 12204081, JP 12302765, JP 6327488 and JP 98001467; in European patent applications EP 937 723, EP 937 711, EP 874 629, EP 842 941, EP 728 758, EP 540 051, EP 419 099, EP 686 642, EP 1 016 663 and EP 529 715; and in German patent applications Nos. DE 19845153, DE 19835950, DE 19743435, DE 19829964, DE 19834751, DE 19839499, DE19900355, DE19900471 and DE 19530996, the specific and generic disclosures in all of which documents are hereby incorporated by reference.

Factor Xa inhibitors that may be mentioned also include those disclosed in international patent applications WO 96/10022, WO 97/28129, WO 97/29104, WO 98/21188, WO 99/06371, WO 99/57099, WO 99/57112, WO 00/47573, WO 00/78749, WO 99/09027 and WO 99/57113, the specific and generic disclosures in all of which documents are hereby incorporated by reference, as well as 4-{4-[4-(5-chloroindol-2-ylsulfonyl) piperazine-1-carbonyl]phenyl}pyridine-1-oxide and pharmaceutically-acceptable derivatives thereof, which may be prepared according to the method described in Example 1 below.

Preferred Factor Xa inhibitors include antistatin, tick anticoagulant protein and those known as SQ-311 and SQ-315 (see international patent application WO 98/57951); SN-292 (see international patent application WO 98/28282); SN-429 and SN 116 (see international patent application WO 98/28269); RPR-208707 (see international patent application WO 98/25611 at Example 48); XU-817 (see international patent application WO 98/01428); SF-324 and SF-303 (see international patent application WO 97/23212); YM 60828 (see international patent application WO 96/16940 at Example 75); FACTOREX (see US patent No. 5,783,421); SF-324 (see European patent application EP 874 629); DX9065A (see European patent application EP 540 051 at Example 39); 1-(4-amidinobenzyl)-4-(6-chloronaphthalene-2-ylsulfonyl)piperazin-2-one (see JP 12204081 at Example 2); M55555 (see international patent application WO 99/33805 at Example 39); DPC423 (1-(3-amidinophenyl)-2-(2'-aminosulfonyl[1,1'-biphenyl]-4-ylaminocarbonyl)-4-bromopyrrole, see international patent application WO 98/28269); 3-(3,5-difluoro-6-[3-(4,5-dihydro-1-methylimidazol-2-yl)phenoxy]-4-[2,3-dihydroxypropoxy]-pyridin-2-yloxy)-4-hydroxybenzamidine (see international patent application WO 00/31068); ZK-807834 (see international patent application WO 97/29067); 1,4-diaza-4-(6-chloronaphthalene-2-ylsulfonyl)-6-(methoxymethyl)-7-oxa-1'-(pyridin-4-yl)spiro[bicyclo[4.3.0]-nonane-8,4'-piperidine]-2-one (see international patent application WO 01/02397); (S)-1-(4-aminoquinazolin-7-ylmethyl)-4-[2-(5-chlorothien-2-yl-oxy)acetyl-3-methoxymethylpiperazin-2-one (see international patent application WO 00/32590); 3-(2-[4-(2-aminosulfonylphenyl)benzoylamino]phenoxy)benzamidine (see international patent application WO 01/19788); and 4-(2-[4-(5-chloroindol-2-ylsulfonyl)-2-(pyrrolidin-1-ylcarbonylmethyl)piperazin-1-ylcarbonyl]thiazol-5-yl)pyridine N-oxide (see Japanese patent application No. JP 12143623); as well as the compounds of Example 7 of international patent application WO 98/21188, of Examples 3 and 6 of WO 99/57113, of Example 6 of international patent application WO 00/78747, of Examples 188, 211 and 167 of US patent No. 6,080,767, of Examples 40, 54 and 55 of international patent application WO 99/33805, of Examples 5, 6, 8, 9, 10, 11, 12, 13, 15, 16 and 17 of international patent application WO 01/05784, of Examples 6, 7, 8, 9, 10, 11, 12, 13, 14, 15, 16, 17, 22, 23, 25, 26, 28, 29, 30, 31, 32, 33, 34, 38, 39, 40, 41, 42 and 43 of international patent application WO 01/12600, and of Examples 802 and 877 of international patent application WO 00/35886.

The term "condition where anticoagulant therapy is indicated" will be understood by those skilled in the art to include the following:

The treatment and/or prophylaxis of thrombosis and hypercoagulability in blood and/or tissues of animals including man. It is known that hypercoagulability may lead to thrombo-embolic diseases. Conditions associated with hypercoagulability and thrombo-embolic diseases which may be mentioned include inherited or acquired activated protein C resistance, such as the factor V-mutation (factor V Leiden), and inherited or acquired deficiencies in antithrombin III, protein C, protein S, heparin cofactor II. Other conditions known to be associated with hypercoagulability and thrombo-embolic disease include circulating antiphospholipid antibodies (Lupus anticoagulant), homocysteinemi, heparin induced thrombocytopenia and defects in fibrinolysis, as well as coagulation syndromes (e.g. disseminated intravascular coagulation (DIC)) and vascular injury in general (e.g. due to surgery).

The treatment of conditions where there is an undesirable excess of thrombin without signs of hypercoagulability, for example in neurodegenerative diseases such as Alzheimer's disease.

Particular disease states which may be mentioned include the therapeutic and/or prophylactic treatment of venous thrombosis (e.g. DVT) and pulmonary embolism, arterial thrombosis (e.g. in myocardial infarction, unstable angina, thrombosis-based stroke and peripheral arterial thrombosis), and systemic embolism usually from the atrium during atrial fibrillation or from the left ventricle after transmural myocardial infarction, or caused by congestive heart failure; prophylaxis of reocclusion (ie thrombosis) after thrombolysis, percutaneous trans-luminal angioplasty (PTA) and coronary bypass operations; the prevention of rethrombosis after microsurgery and vascular surgery in general.

Further indications include the therapeutic and/or prophylactic treatment of disseminated intravascular coagulation caused by bacteria, multiple trauma, intoxication or any other mechanism; anticoagulant treatment when blood is in contact with foreign surfaces in the body such as vascular grafts, vascular stents, vascular catheters, mechanical and biological prosthetic valves or any other medical device; and anticoagulant treatment when blood is in contact with medical devices outside the body such as during cardiovascular surgery using a heart-lung machine or in haemodialysis; the therapeutic and/or prophylactic treatment of idiopathic and adult respiratory distress syndrome, pulmonary fibrosis following treatment with radiation or chemotherapy, septic shock, septicemia, inflammatory responses, which include, but are not limited to, edema, acute or chronic atherosclerosis such as coronary arterial disease and the formation of atherosclerotic plaques, cerebral arterial disease, cerebral infarction, cerebral thrombosis, cerebral embolism, peripheral arterial disease, ischaemia, angina (including unstable angina), reperfusion damage, restenosis after percutaneous trans-luminal angioplasty (PTA) and coronary artery bypass surgery.

Preferred conditions include thrombosis.

In accordance with the invention, melagatran, prodrugs thereof, Factor Xa inhibitors and salts or solvates of either, may be administered orally, intravenously, subcutaneously, buccally, rectally, dermally, nasally, tracheally, bronchially, topically, by any other parenteral route, or via inhalation, in the form of a pharmaceutical preparation comprising melagatran and/or Factor Xa inhibitor in a pharmaceutically-acceptable dosage form. Depending on the disorder, and the patient, to be treated, as well as the route of administration, the compositions may be administered at varying doses.

Preferred modes of delivery are systemic. For melagatran and salts or solvates thereof, preferred modes of administration are parenteral, more preferably intravenous, and especially subcutaneous. For prodrugs of melagatran, preferred modes of administration are oral.

In the therapeutic treatment of mammals, and especially humans, melagatran, Factor Xa inhibitors, and derivatives of either will generally be administered as a pharmaceutical formulation in admixture with a pharmaceutically-acceptable adjuvant, diluent or carrier, which may be selected with due regard to the intended route of administration and standard pharmaceutical practice.

Suitable formulations for use in administering melagatran and salts, solvates or prodrugs thereof are described in the literature, for example as described in *inter alia* international patent applications WO 94/29336, WO 96/14084, WO 96/16671, WO 97/23499, WO 97/39770, WO 97/45138, WO 98/16252, WO 99/27912, WO 99/27913, WO 00/12043 and WO 00/13671, the disclosures in which documents are hereby incorporated by reference:

Similarly, suitable formulations for use in administering Factor Xa inhibitors and salts or solvates thereof are described in the literature, for example as described in the prior art documents relating to Factor Xa inhibitors that are mentioned hereinbefore, the disclosures in which documents are hereby incorporated by reference. Otherwise, the preparation of suitable formulations, and in particular combined preparations including both melagatran/salt, solvate or prodrug and Factor Xa inhibitor/salt or solvate may be achieved non-inventively by the skilled person using routine techniques.

The amounts of melagatran, prodrug thereof, Factor Xa inhibitor, or salt or solvate of either, in the respective formulation(s) will depend on the severity of the condition, and on the patient, to be treated, as well as the compound (s) which is/are employed, but may be determined non-inventively by the skilled person.

Suitable doses of melagatran, prodrug thereof, Factor Xa inhibitors and salt or solvate of either, in the therapeutic and/or prophylactic treatment of mammalian, especially human, patients may be determined routinely by the medical practitioner or other skilled person, and include the respective doses discussed in the prior art documents relating to melagatran (or salts, solvates or prodrugs) thereof, and to Factor Xa inhibitors, that are mentioned hereinbefore, the disclosures in which documents are hereby incorporated by reference.

In the case of melagatran, suitable doses of active compound, prodrugs and salts or solvates thereof, in the therapeutic and/or prophylactic treatment of mammalian, especially human, patients include those which give a mean plasma concentration of up to 5 $\mu$mol/L, for example in the range 0.001 to 5 $\mu$mol/L over the course of treatment of the relevant condition. Suitable doses may thus be in the range 0.1 mg once daily to 25 mg three times daily, and/or up to 100 mg infused parenterally over a 24 hour period, for melagatran, and in the range 0.1 mg once daily to 100 mg three times daily for prodrugs of melagatran including those specifically mentioned herein.

In the case of Factor Xa inhibitors, suitable doses for therapeutic or prophylactic purposes are in the range such that, for example, 10 to 500 mg is received, given if required in divided doses. In general lower doses will be administered when a parenteral route is employed, for example a dose for intravenous administration in the range, for example, 1 to 50 mg will generally be used. At continuous infusion, a dose of between 0.1 and 5 mg/kg/hour will generally be used.

In any event, the physician, or the skilled person, will be able to determine the actual dosage which will be most suitable for an individual patient, which is likely to vary with the condition that is to be treated, as well as the age, weight, sex and response of the particular patient to be treated. The above-mentioned dosages are exemplary of the average case; there can, of course, be individual instances where higher or lower dosage ranges are merited, and such are within the scope of this invention.

When separate formulations are administered, the sequence in which the formulations comprising melagatran (or salt, solvate or prodrug thereof), and Factor Xa inhibitor (or salt or solvate thereof), may be administered (i.e. whether, and at what point, sequential, separate and/or simultaneous administration takes place) may be determined by the physician or skilled person. For example, the sequence may depend upon many factors that will be evident to the skilled person, such as whether, at any time during the course or period of treatment, one or other of the formulations cannot be administered to the patient for practical reasons (e.g. the patient is unconscious and thus unable to take an oral formulation comprising either melagatran or Factor Xa inhibitor).

The method described herein may have the advantage that, in the treatment of conditions where anticoagulant therapy is indicated, it may be more convenient for the physician and/or patient than, be more efficacious than, be less toxic than, have a broader range of activity than, be more potent than, produce fewer side effects than, or that it may have other useful pharmacological properties over, similar methods known in the prior art for the treatment of such conditions.

The invention is illustrated, but in no way limited, by the following examples with reference to the figures in which:

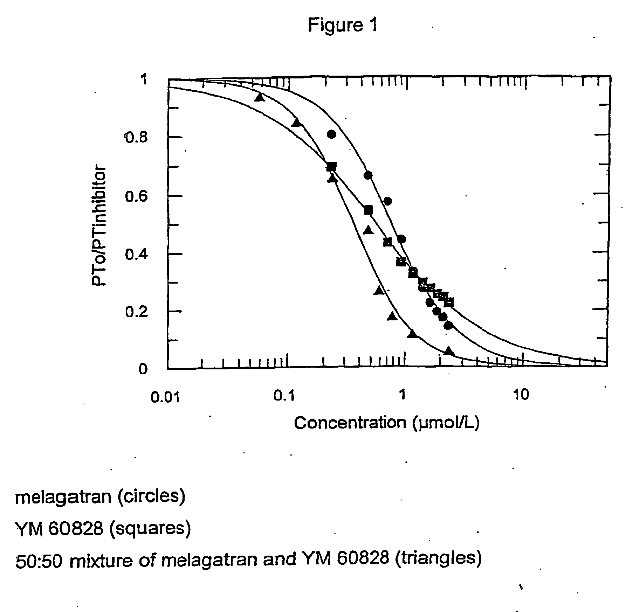

Figure 1 illustrates dose-response curves for melagatran (circles), the Factor Xa inhibitor YM 60828 (squares), and a 50/50 mixture of melagatran and YM 60828 (triangles) in a prothrombin time clotting assay.

Example 1

4-{4-[4-(5-Chloroindol-2-ylsulfonyl) piperazine-1-carbonyl]phenyl}-pyridine-1-oxide

To a stirred suspension of 1-(5-chloroindol-2-ylsulfonyl)-4-[4-(4-pyridyl)benzoyl] piperazine (500 mg, 1.04 mmol) in dichloromethane (25 mL) was added 3-chloroperoxybenzoic acid (640 mg of 70-75 % strength, 2.6 mmol, 2.5 mol eq.) and the reaction stirred for 2 hours at ambient temperature, at which time use of thin layer chromatography indicated complete reaction. The reaction mixture was washed sequentially with sodium metabisulphite solution (2 x 20 mL of 1M) and brine, and then dried (phase-separating paper). Evaporation gave crude product as a brown solid; crystallisation from ethanol gave 4-{4-[4-(5-chloroindol-2-ylsulfonyl) piperazine-1-carbonyl]phenyl}pyridine-1-oxide as a pale yellow crystalline solid.

$^1$H NMR ($d_6$-DMSO): 3.0-3.3 (broad s, 4H), 3.4-3.9 (broad d, 4H), 7.0 ppm (s, 1H), 7.35 (d, 1H), 7.5 (m, 3H), 7.8 (m, 5H), 8.3 (d,2H), 12.4 (s,1H)

MS (M+H)$^+$ 495/497

mp 265-267°C

The preparation of 1-(5-chloroindol-2-ylsulfonyl)-4-[4-(4-pyridyl)benzoyl] piperazine is described in Example 3 of international patent application WO 99/57113.

Example 2

Synergistic Effect of Melagatran and a Factor Xa Inhibitor in Prothrombin Time Clotting Assay

Human blood from healthy volunteers, both men and women, was collected in 50 mL conical plastic tubes containing 5 mL of 0.13 mol/L of buffered tri-sodium citrate. The blood was immediately chilled on ice and centrifuged at 2000 x g for 20 minutes. Platelet poor plasma was then divided into fractions of 10 mL each and "spiked" with melagatran or with YM 60828 (a direct Factor Xa inhibitor; see Example 75 of international patent application WO 96/16940) to a final concentration of 2.33 $\mu$mol/L. The melagatran solution was then mixed with YM 60828 in serial proportions (9:1, 8:2, 7:3 etc.) as indicated in Table 1.

Table 1

| Melagatran $\mu$mol/L | YM 60828 $\mu$mol/L | Mixture $\mu$mol/L |
|---|---|---|
| 2.33 | 0.00 | 2.33 |
| 2.10 | 0.23 | 2.33 |
| 1.86 | 0.47 | 2.33 |
| 1.63 | 0.70 | 2.33 |
| 1.40 | 0.93 | 2.33 |
| 1.17 | 1.17 | 2.33 |
| 0.93 | 1.40 | 2.33 |
| 0.70 | 1.63 | 2.33 |
| 0.47 | 1.86 | 2.33 |
| 0.23 | 2.10 | 2.33 |
| 0.00 | 2.33 | 2.33 |

100 $\mu$L of human plasma was pre-warmed to 37°C for 180 seconds before coagulation was initiated by the addition of 200 $\mu$L pre-warmed assay reagent (Thromboplastin-S, ISI 1.1, Biopool Umeå, Sweden). The prothrombin time (PT) was determined on a mechanical coagulometer, coupled to a CR-A calculator, CR 10 printer and a PR60 heat block (KC 10A Amelung, Germany). Each clotting test was performed in duplicate. The prothrombin time for melagatran and YM 60828 (2.33 $\mu$mol/L) was 95.2 and 64.9 seconds, respectively, whereas all mixtures of these inhibitors (still at a total concentration of 2.33 $\mu$mol/L) showed a surprisingly prolonged PT with a maximum around 300 to 310 seconds when the inhibitors were mixed at a proportion of 60 - 40 % thrombin inhibitor and 40 - 60 % factor Xa inhibitor. All results are shown in Table 2.

**Table 2**

| Melagatran $\mu$mol/L | YM 60828 $\mu$mol/L | Mixture $\mu$mol/L | PT (sec) seconds |
|---|---|---|---|
| 2.33 | 0.00 | 2.33 | 95.2 |
| 2.10 | 0.23 | 2.33 | 203.8 |
| 1.86 | 0.47 | 2.33 | 264.4 |
| 1.63 | 0.70 | 2.33 | 300.0 |
| 1.40 | 0.93 | 2.33 | 311.3 |
| 1.17 | 1.17 | 2.33 | 311.8 |
| 0.93 | 1.40 | 2.33 | 306.0 |
| 0.70 | 1.63 | 2.33 | 284.2 |
| 0.47 | 1.86 | 2.33 | 243.4 |
| 0.23 | 2.10 | 2.33 | 185.5 |
| 0.00 | 2.33 | 2.33 | 64.9 |

In order to test whether this is a "true" synergistic effect or not, full dose-response curves for each inhibitor as well as for a 50:50 mixture of melagatran and YM 60828 were constructed. Each inhibitor was tested at concentrations ranging from 0 to 2.33 $\mu$mol/L, as shown in Tables 3 (melagatran and YM 60828) and 4 (a 50:50 mixture of melagatran and YM 60828).

Table 3

| Plasma conc. | Prothrombin time (sec) | |
|---|---|---|
| $\mu$mol/L | melagatran | YM 60828 |
| 0.00 | 14.4 | 14.9 |
| 0.23 | 18.1 | 21.6 |
| 0.47 | 21.8 | 27.7 |
| 0.70 | 25.4 | 34.4 |
| 0.93 | 32.6 | 41.8 |
| 1.17 | 43.2 | 46.8 |
| 1.40 | 53.8 | 52.1 |
| 1.63 | 65.3 | 55.5 |
| 1.86 | 76.3 | 60.2 |
| 2.10 | 87.2 | 62.1 |

Table 4

| Plasma concentration ($\mu$mol/L) | | | |
|---|---|---|---|
| Total | Melagatran | YM 60828 | Prothrombin time |
| 0.00 | 0.00 | 0.00 | 14.2 |
| 0.058 | 0.029 | 0.029 | 15.2 |
| 0.117 | 0.058 | 0.058 | 17.0 |
| 0.233 | 0.117 | 0.117 | 21.7 |
| 0.466 | 0.233 | 0.233 | 30.2 |
| 0.583 | 0.291 | 0.291 | 53.8 |
| 0.777 | 0.388 | 0.388 | 85.4 |
| 1.165 | 0.583 | 0.583 | 134.7 |

Table continued

| | Plasma concentration ($\mu$mol/L) | | |
|---|---|---|---|
| Total | Melagatran | YM 60828 | Prothrombin time |
| 2.330 | 1.165 | 1.165 | 300.0 |

In order to determine the relative potency of each inhibitor and a 50:50 mixture thereof, the concentration required to double the prothrombin time ($IC_{50}$) was determined as follows: The ratio of prothrombin time in control plasma ($PT_0$) to the prothrombin time in the presence of different inhibitor concentrations ($PT_{inhibitor}$) were plotted against the inhibitor concentration (log transformed) and fitted to sigmoidal dose-response curves from which $IC_{50}$ values were calculated according to the equation:

$$y = a/[1+(x/IC_{50})^s]$$

where y= $(PT_0)/(PT_{mela})$ with a range from 1 to 0; a = maximal range i.e. 1, when $PT_{mela}$ is equal to $PT_0$; s = the slope of the dose-response curve, x = log concentration of inhibitor, $IC_{50}$ = the concentration of melagatran that doubles the prothrombin time. The calculations were processed on a PC using the software program GraFit® Erithacus Version 3.0 (Robin Leatherbarrow, Imperial Collage of Science, Technology & Medicine, London).

The dose-response curves for melagatran, YM 60828, and a 50:50 mixture of these inhibitors are shown in Figure 1 from which $IC_{50}$ values were calculated to be 0.74, 0.54 and 0.34 $\mu$mol/L, respectively.

In order to test if the lower $IC_{50}$ value for the mixture, as compared to both melagatran and YM 60828 alone reflects a true synergistic effect, the results were applied to an equation for synergy, as defined by Beerenbaum:

$$dose\ A/A_E + dose\ B/B_E < 1$$

in which $A_E$ and $B_E$ are equipotent concentrations of melagatran and YM 60828 (i.e. the $IC_{50}$ values for these compounds which were 0.74 and 0.54 $\mu$mol/L. respectively). Dose A and B are the concentration of melagatran and YM 60828 in the 50:50 mixture which gave an $IC_{50}$ value of 0.34 $\mu$mol/L, i.e. A and B is 0.17 $\mu$mol/L. Entering these constants into the above equation results in a value of 0.54, which demonstrates that the synergy criterion is fulfilled, and melagatran and YM 60828 exert a true synergistic effect *in vitro* when they are tested in a prothrombin time clotting assay.

**Claims**

1. A combination product comprising:

   (A) melagatran or a salt, solvate, or prodrug thereof, wherein the prodrug is of the formula

   $R^1O_2C\text{-}CH_2\text{-}(R)Cgl\text{-}Aze\text{-}Pab\text{-}OH,$

   wherein $R^1$ represents $C_{1-10}$ alkyl or benzyl and the OH group replaces one of the amidino hydrogens in Pab; and
   (B) a Factor Xa inhibitor or a salt or solvate thereof,

   wherein each of components (A) and (B) is formulated in admixture with a pharmaceutically-acceptable adjuvant, diluent or carrier.

2. A combination product as claimed in Claim 1 which comprises a pharmaceutical formulation including melagatran, the salt, the solvate or the prodrug thereof, and the Factor Xa inhibitor, the salt or the solvate thereof, and a pharmaceutically-acceptable adjuvant, diluent or carrier.

3. A combination product as claimed in Claim 1 which comprises a kit of parts comprising components:

   (a) a pharmaceutical formulation including melagatran, the salt, the solvate or the prodrug thereof, in admixture with a pharmaceutically-acceptable adjuvant, diluent or carrier; and
(b) a pharmaceutical formulation including the Factor Xa inhibitor, the salt or the solvate thereof, in admixture with a pharmaceutically-acceptable adjuvant, diluent or carrier,

which components (a) and (b) are each provided in a form that is suitable for administration in conjunction with the other.

4. A kit of parts as claimed in Claim 3, wherein components (a) and (b) are suitable for sequential, separate and/or simultaneous use in the treatment of a condition where anticoagulant therapy is indicated.

5. A combination product as claimed in any one of Claims 1 to 4, wherein in the prodrug,
$R^1$ represents linear or branched $C_{1-6}$ alkyl.

6. A combination product as claimed in Claim 5, wherein $R^1$ represents methyl, ethyl, *n*-propyl, *i*-propyl or *t*-butyl.

7. A combination product as claimed in Claim 6, wherein $R^1$ represents ethyl.

8. A method of making a kit of parts as defined in any one of Claims 3 to 7, which method comprises bringing a component (a), as defined in any one of Claims 3 to 7, into association with a component (b), as defined in any one of Claims 3 to 7, thus rendering the two components suitable for administration in conjunction with each other.

9. A kit of parts comprising:

   (I) one of components (a) and (b) as defined in any one of Claims 3 to 7; together with
   (II) instructions to use that component in conjunction with the other of the two components.

10. The use of a combination product as defined in any one of Claims 1 to 7 or a kit of parts as defined in Claim 9 for the manufacture of a medicament for the treatment or prophylaxis of a condition where anticoagulant therapy is indicated.

11. The use of melagatran or a salt, solvate, or prodrug thereof, wherein the prodrug is of the formula

   $$R^1O_2C\text{-}CH_2\text{-}(R)Cgl\text{-}Aze\text{-}Pab\text{-}OH,$$

   wherein $R^1$ represents $C_{1-10}$ alkyl or benzyl and the OH group replaces one of the amidino hydrogens in Pab and a Factor Xa inhibitor or a salt or solvate thereof for the manufacture of a medicament for the treatment or prophylaxis of a condition where anticoagulant therapy is indicated.

**Patentansprüche**

1. Kombinationsprodukt, umfassend:

   (A) Melagatran oder ein Salz, Solvat oder Prodrug davon, wobei das Prodrug die Formel

   $$R^1O_2C\text{-}CH_2\text{-}(R)\ Cgl\text{-}Aze\text{-}Pab\text{-}OH$$

   aufweist, in der $R^1$ $C_{1-10}$-Alkyl oder Benzyl darstellt und die OH-Gruppe ein Amidino-Hydrogen in Pab ersetzt; und
   (B) einen Faktor-Xa-Hemmer oder ein Salz oder Solvat davon;

   worin jeder der Bestandteile (A) und (B) als Gemisch mit einem pharmazeutisch annehmbaren Adjuvans, Verdünnungsmittel oder Träger formuliert wird.

2. Kombinationsprodukt nach Anspruch 1, das eine pharmazeutische Formulierung umfasst, die Melagatran, das Salz, das Solvat oder das Prodrug davon, und den Faktor-Xa-Hemmer, das Salz oder das Solvat davon und ein pharmazeutisch annehmbares Adjuvans, Verdünnungsmittel oder einen Träger enthält.

3. Kombinationsprodukt nach Anspruch 1, das ein Set aus Teilen mit folgenden Bestandteilen umfasst:

(a) eine pharmazeutische Formulierung, die Melagatran, das Salz, das Solvat oder das Prodrug davon im Gemisch mit einem pharmazeutisch annehmbaren Adjuvans, Verdünnungsmittel oder Träger enthält; und
(b) eine pharmazeutische Formulierung, die den Faktor-Xa-Hemmer, das Salz oder das Solvat davon im Gemisch mit einem pharmazeutisch annehmbaren Adjuvans, Verdünnungsmittel oder Träger enthält,

wobei die Bestandteile (a) und (b) jeweils in einer Form vorliegen, die zur Verabreichung in Verbindung mit dem anderen Bestandteil geeignet ist.

4. Set aus Teilen nach Anspruch 3, wobei die Bestandteile (a) und (b) für die sequentielle, getrennte und/oder gleichzeitige Verwendung bei der Behandlung einer Erkrankung, bei der eine gerinnungshemmende Therapie angezeigt ist, geeignet sind.

5. Kombinationsprodukt nach einem der Ansprüche 1 bis 4, worin im Prodrug $R^1$ lineares oder verzweigtes $C_{1-6}$-Alkyl darstellt.

6. Kombinationsprodukt nach Anspruch 5, worin $R^1$ Methyl, Ethyl, n-Propyl, i-Propyl oder t-Butyl darstellt.

7. Kombinationsprodukt nach Anspruch 6, worin $R^1$ Ethyl darstellt.

8. Verfahren zur Herstellung eines Sets aus Teilen wie in einem der Ansprüche 3 bis 7 definiert, wobei bei dem Verfahren ein Bestandteil (a) wie in einem der Ansprüche 3 bis 7 definiert mit einem Bestandteil (b) wie in einem der Ansprüche 3 bis 7 definiert zusammen gebracht wird, so dass die beiden Bestandteile gemeinsam verabreicht werden können.

9. Set aus Teilen, umfassend:

(I) einen der Bestandteile (a) und (b) wie in einem der Ansprüche 3 bis 7 definiert; zusammen mit
(II) Anweisungen zur Verwendung dieses Bestandteils zusammen mit dem anderen der beiden Bestandteile.

10. Verwendung eines Kombinationsprodukts wie in einem der Ansprüche 1 bis 7 definiert oder eines Sets aus Teilen wie in Anspruch 9 definiert zur Herstellung eines Medikaments zur Behandlung oder Prophylaxe einer Erkrankung, bei der eine gerinnungshemmende Therapie angezeigt ist.

11. Verwendung von Melagatran oder eines Salzes, Solvats oder Prodrugs davon, wobei das Prodrug die Formel

$$R^1O_2C-CH_2- \text{(R) Cgl-Aze-Pab-OH}$$

aufweist, in der $R^1$ $C_{1-10}$-Alkyl oder Benzyl darstellt und die OH-Gruppe ein Amidino-Hydrogen in Pab ersetzt, und eines Faktor-Xa-Hemmers oder eines Salzes oder Solvats davon zur Herstellung eines Medikaments zur Behandlung oder Prophylaxe einer Erkrankung, bei der eine gerinnungshemmende Therapie angezeigt ist.

## Revendications

1. Produit combiné comprenant :

(A) du mélagatran ou un sel, un solvate ou un précurseur de médicament de celui-ci, le précurseur de médicament ayant la formule

$$R^1O_2C-CH_2- \text{(R) Cgl-Aze-Pab-OH}$$

dans laquelle $R^1$ représente un groupe alkyle en $C_1$ à $C_{10}$ ou benzyle et le groupe OH remplace un des hydrogènes amidino dans Pab et
(B) un inhibiteur du facteur Xa ou un sel ou un solvate de celui-ci,
dans lequel chacun des composants (A) et (B) est formulé en mélange avec un adjuvant, un diluant ou un support pharmaceutiquement acceptable.

2. Produit combiné selon la revendication 1, qui comprend une formulation pharmaceutique comprenant du mélagatran, le sel, le solvant ou le précurseur de médicament de celui-ci et l'inhibiteur du facteur Xa, le sel ou le solvate de celui-ci et un adjuvant, un diluant ou un support pharmaceutiquement acceptable.

3. Produit combiné selon la revendication 1, qui comprend un kit de parties, comprenant les composants

(a) une formulation pharmaceutique comprenant du mélagatran, le sel, le solvate ou le précurseur de médicament de celui-ci, en mélange avec un adjuvant, un diluant ou un support pharmaceutiquement acceptable et
(b) une formulation pharmaceutique comprenant l'inhibiteur du facteur Xa, le sel ou le solvate de celui-ci, en mélange avec un adjuvant, un diluant ou un support pharmaceutiquement acceptable

les composants (a) et (b) étant chacun proposés dans une forme qui est appropriée pour une administration combinée avec l'autre.

4. Kit de parties selon la revendication 3, dans lequel les composants (a) et (b) conviennent pour une utilisation séquentielle, séparée et/ou simultanée dans le traitement d'un état où une thérapie par un anticoagulant est indiquée.

5. Produit combiné selon l'une quelconque des revendications 1 à 4, dans lequel, dans le précurseur de médicament, $R^1$ représente un groupe alkyle en $C_1$ à $C_6$ linéaire ou ramifié.

6. Produit combiné selon la revendication 5, dans lequel $R^1$ représente un groupe méthyle, éthyle, n-propyle, i-propyle ou t-butyle.

7. Produit combiné selon la revendication 6, dans lequel $R^1$ représente un groupe éthyle.

8. Procédé pour préparer un kit de parties selon l'une quelconque des revendications 3 à 7, ledit procédé comprenant la combinaison d'un composant (a) selon l'une quelconque des revendications 3 à 7 avec un composant (b) selon l'une quelconque des revendications 3 à 7, rendant ainsi les deux composants appropriés pour l'administration combinée les uns avec les autres.

9. Kit de parties comprenant

(I) un des composants (a) et (b) selon l'une quelconque des revendications 3 à 7 avec
(II) des instructions pour utiliser ce composant en combinaison avec l'autre des deux composants.

10. Utilisation d'un produit combiné selon l'une quelconque des revendications 1 à 7 ou d'un kit de parties selon la revendication 9 pour la préparation d'un médicament destiné au traitement ou à la prophylaxie d'un état dans lequel une thérapie par un anticoagulant est indiquée.

11. Utilisation de mélagatran ou d'un sel, d'un solvate ou d'un précurseur de médicament de celui-ci, le précurseur de médicament ayant la formule

$$R^1O_2C\text{-}CH_2\text{-}(R)Cgl\text{-}Aze\text{-}Pab\text{-}OH$$

dans laquelle $R^1$ représente un groupe alkyle en $C_1$ à $C_{10}$ ou benzyle et le groupe OH remplace un des hydrogènes amidino dans Pab et d'un inhibiteur du facteur Xa ou d'un sel ou d'un solvate de celui-ci pour la préparation d'un médicament pour le traitement ou la prophylaxie d'un état où une thérapie avec un anticoagulant est indiquée.

Figure 1

melagatran (circles)

YM 60828 (squares)

50:50 mixture of melagatran and YM 60828 (triangles)